Europäisches Patentamt

European Patent Office

Office européen des brevets

(19)

(11) Publication number : **0 589 612 A2**

(12) **EUROPEAN PATENT APPLICATION**

(21) Application number : **93307276.1**

(22) Date of filing : **15.09.93**

(51) Int. Cl.⁵ : **G01N 33/537**

(30) Priority : **16.09.92 US 945690**

(43) Date of publication of application :
**30.03.94 Bulletin 94/13**

(84) Designated Contracting States :
**AT BE CH DE ES FR GB IT LI NL SE**

(71) Applicant : **NAVIX INC.**
**96 Natalie Way**
**Camarillo, California 93010 (US)**

(72) Inventor : **Hepp, Jozsef**
**Bakonyi u. 25**
**H-2030 Erd (HU)**
Inventor : **Lengyel, Zsolt**
**Torok u. 2**
**H-1023 Budapest (HU)**
Inventor : **Petho, Arpad**
**Vizimolnar u. 33**
**H-1031 Budapest (HU)**
Inventor : **Gorog, Gyorgy**
**Csillag u. 8**
**H-1193 Budapest (HU)**

(74) Representative : **Harvey, David Gareth et al**
**Graham Watt & Co. Riverhead**
**Sevenoaks Kent TN13 2BN (GB)**

(54) **Label trapping, nonseparation binding assay.**

(57) A method for determining the amount of analyte in a sample which comprises a first step of providing a first assay compartment and a second assay compartment separated from the first assay compartment by a barrier permeable to a signal providing binding reagent-label conjugate, the barrier e.g. being a porous membrane or gel ; a second step of combining an analyte containing sample and binding reagent-label conjugate capable complex of binding to the analyte to form alone or in combination with a second binding reagent a specific binding pair complex of a size sufficiently large to prevent passage through the barrier, said binding reagent-label conjugate being provided in an excess of the amount required to bind with all of the analyte ; a third step of incubating the mixture to allow the analyte to bind to the binding reagent-label conjugate to form the specific binding pair comprising the analyte and the binding reagent-label conjugate and for unbound binding reagent-label conjugate to pass through the barrier into the second compartment ; a fourth step of entrapping and modifying the unbound binding reagent-label conjugate in the second compartment to enable detection of a signal from specific binding pair as a measure of the amount of analyte bound in the specific binding pair ; and a fifth step of measuring the detectable signal.

Fig. 1

This invention relates to binding assays of analytes, materials and assay arrangements used therein, more particularly to nonseparation binding assays such as immunoassays, DNA-probe, RNA-probe and other nucleic acid detection assays and receptor-ligand binding assays.

There are a large number of methods with the purpose of detecting certain organic compounds and determining their concentration in a sample.

An important, dynamically developing group of these methods includes binding assays.

Many analytical procedures applied in practice involve a specific binding pair (BP). The analyte to be determined (A) is a member of this BP. The principle of these methods is that A is capable of specifically binding to the other member of BP, namely a binding reagent, (BR), and the result of this binding, that is, the formation of the BP complex is detected some way. Analytical procedures of this kind are called binding assays. Characteristic groups of binding assays are immunoassays, DNA probe assays, and receptor-ligand binding assays. During the execution of a binding assay the occurrence of binding can be detected by physical methods like precipitation, alterations of optical characteristics etc. Such methods are detailed in Rose, N.R., Friedman, H. and Fahey, J.L. (eds): Manual of clinical Laboratory Immunology, American Society for Microbiology, Washington, D.C., 1986, incorporated herein by reference.

Preferably, however, another component capable of emitting a signal is employed; this component is termed a label (L). The presence of L makes possible or easier the detection of BP complex formation. Labels can be molecules labelled with radioisotopes, enzymes, fluorescent or luminescent molecules etc. Such labels and the methods suitable for coupling such labels to assay components are detailed in Lange, D. (ed): Antibodies. A Laboratory Manual. Cold Spring Harbor Laboratory, 1988, incorporated herein by this reference.

L may be attached to BR, A, or both, or the already formed BP complex, or an analog of BR and/or A. A notable group of procedures including the present invention is characteristic by the attachment of L to BR to form a labeled binding reagent (BR-L). These methods can be divided in two large groups: in one, the BR-L conjugates which did not react with A are removed before these unreacted BR-L conjugates remain in the assay. The latter are often termed homogeneous or nonseparation assays.

Examples of various heterogeneous assays can be seen in the following patent specifications and patent applications: EP 272,691, US 4,808,521, US 4,299,916, US 4,614,712, US 4,688,619, UK 2,052,057, UK 2,065,302, US 4,725,536, US 4,818,680, US 4,775,619, EP 153,873, EP 164,876, EP 198,662, EP 141,547 each incorporated herein by this reference. These procedures are characterized by the coupling of A-BR-L complexes to a solid carrier, and the BR-L conjugates not bound to A are removed by phase separation.

Homogeneous (nonseparating) binding assays are described in the following patent specifications and applications: US 4,629,690, US 4,323,647, US 4,640,898, US 4,629,688, US 4,668,619, US 4,510,240, US 4,446,233, EP 070,685, EP 149,338, EP 231,495, EP 425,206, US 4,307,070, US 4,288,425, each incorporated herein by this reference.

The following examples of homogeneous (nonseparating) assays can be considered as the most relevant prior art:

The patents No. US 4,687,735, US 4,633,278, EP 122,059 each incorporated herein by this reference are based on various realizations of the channelling principle: upon formation of the A-BR complexes two entities come into spatial proximity and in consequence of this the signal emitted by them is measurably altered.

In particular, Litman et al (Anal. Biochem. 106, 223, 1980), incorporated herein by this reference, describe an enzyme channelling immunoassay that enhances the channelling efficiency by immobilizing one member of a specific binding pair into porous agarose beads.

According to the procedure invented by Litman et al. (US 4,275,149 and US 4,374,925 incorporated herein by this reference) one member of the BP is coupled to a particle that provides for L an environment in the bulk of the liquid; and signal emitted by L in this environment is different from the signal emitted by the unbound L in the bulk of the liquid.

The patent WO 8,902,406 (GenProbe) incorporated herein by reference discloses a new homogeneous assay, in which assay the formation of A-BR-L complex is not accompanied by any alteration of the emitted signal, but the signal emitting capability of the BR-L conjugates which remained unbound (in excess) is destroyed by specific hydrolysis without any phase separation.

Selective membranes have been used to separate molecules of different size. The most common example is dialysis, where a dialysis tube contains the mixture of large and small molecules, and the latter diffuse freely out of the tube into the surrounding liquid while large molecules are retained by the membrane.

This separation process can be facilitated by an electric field, or by centrifugal force or by mechanical pressure as in the case of filtering.

EP 0 589 612 A2

Summary of the Invention:

Methods, compositions and new assay arrangements are provided for the detection without phase separation of an analyte that is a member of a specific binding pair.

The invention provides a novel convenient method for distinguishing the analyte, analyte-analog bound BR-L conjugate molecules, or analyte-second binding reagent bound BR-L conjugated molecules from the excess free BR-L conjugates present in the assay medium, and for altering the signal producing capability of one distinguished portion, which distinction is specially made by size herein. More specifically: a certain portion of the small BR-L molecules becomes part of large and/or small complexes during the course of the assay procedure and the amount of the small or large BR-L complexes is characteristic to the amount of the analyte present in the assayed sample. Consequently, one can measure the signal solely generated by analyte, analyte-second binding reagent bound BR-L conjugate molecules, or analyte-analog bound BR-L conjugates, the measure of which signal is strictly related to the amount of the analyte present in the assayed sample.

Specifically, for the assay, at least two specially separated space compartments are provided. One of them, characterized by adding the sample and the labelled binding reagent (BR-L) and optionally other assay components to it is referred to as Sample Compartment, or first compartment. The other compartment which is separated from the Sample Compartment, is made entirely of some porous or gel forming material having a definite average pore size, is named Modifier Compartment, or second compartment. More specifically, a certain portion of the BR-L conjugates can migrate freely between the compartments, while the remaining BR-L conjugates will be unable to do so.

A further characteristic of the assay is that in the Modifier Compartment the signal producing capability or possibility of all entered free or complexed BR-L conjugates is significantly altered, preferably completely destroyed or enhanced from practically nil to a measurable level. Thus, after an appropriate incubation time, using a suitable assay arrangement, the measured signal can be related to the amount of analyte present.

Without limiting the applicability of the present invention it is mentioned here that the assay method of the present invention is particularly useful in realizing convenient nonseparation immunoassays, DNA-probe, RNA-probe and other nucleic acid detection assays, and receptor-ligand binding assays. Special realizations of the subject method can be used in every field of chemical analysis where detection of an analyte that is a member of a natural or artificial specific binding pair is important, namely: clinical diagnosis, forensic applications, veterinary sciences, medical sciences, biological sciences, environmental pollution testing and other important fields of chemical analysis.

The invention will now be described by way of example only, with reference to the accompanying drawings, in which:

Fig. 1 illustrates the practice of invention where a modified compartment is contained in a sample compartment and the system is used for the detection of large nucleic acid analyte molecules.

Fig. 2 illustrates the practice of the invention where the invention is used for the detection of small bivalent antigenic analyte molecules.

Fig. 3 shows the measured Trypsin active in terms of rate of color change as a function of ferritin-avidin concentration.

DEFINITIONS

As used herein, the following definitions are to be applied:

Analyte (A) - a molecule or a supramolecular entity to be measured. An analyte molecule binds specifically to one or more different binding reagent (BR) molecules.

Analyte analog (A') - a substance similar but not identical to the analyte, capable of binding to the specific binding reagent (BR) with sufficiently high activity.

Specific binding pair (BP) - two different molecules capable of binding to each other with significant higher affinity than to any other molecules present in the assay medium except analyte analogues.

Antibody - a protein molecule originally produced by the immune system of an animal or a human; capable of specifically binding to a compound termed an antigen.

Antigen - a compound provoking a specific immune response producing specific antibodies in an animal or a human.

Signal - a detectable physical or chemical property of a substance or any detectable change in such property.

Label (L) - a substance capable of emitting or inducing a signal or capable of specifically binding a signal producing substance.

Binding reagent (BR) - a substance capable of specifically binding to the analyte and analyte analogues.

3

Binding reagent - label conjugate (BR-L) - covalently conjugated BR and L molecules.

Second binding reagent (SBR) - a substance capable of specifically binding to the analyte together with BR, at the same time.

Binding substance - a substance capable of specifically or nonspecifically binding to the BR-L conjugate without significantly altering its signal emitting capability.

Activator - a substance capable of significantly enhancing the signal generating activity of a label.

Inhibitor - a substance capable of significantly diminishing the signal generating activity of a label.

Large vs. small - all substances and complexes of substances are referred to as large in the present application if they are unable to permeate through the selective barrier separating the assay compartments.

Porous particles - discrete solid particles having small openings (pores), channels or indentations on their surface and in their inner volume.

Capsules, Microcapsules - discrete solid, possibly liquid core particles having a special covering layer on their surface containing small openings (pores).

Specially separated compartments - spatial compartments separated from one another with a selective barrier so that the large entities cannot enter one of these compartments, while the small ones are capable of doing so.

Sample Compartment - the assay compartment where the sample is added.

Modifier Compartment - the assay compartment where a portion of the BR-L conjugate molecules are entrapped and/or their signal producing capability is significantly modified.

Sample - a liquid or a suitably liquified material to be investigated for the presence and concentration of the analyte.

Receptor, ligand - within the scope of the present invention, a receptor is a substance other than an antibody or a nucleic acid, capable of specifically binding another substance termed ligand.

Probe reagent - a substance capable of specifically binding a particular nucleic acid molecule.

Target sequence - a specific nucleic acid sequence present in the analyte molecule which specifically binds the probe reagent.

Selectively permeable membrane or layer - as far as this invention is regarded, selectively permeable means something which is permeable for all small assay components but is impermeable for all large assay components.

## DETAILED DESCRIPTION

An assay method and novel assay arrangements making easy automation possible are provided for the determination of low concentrations of specific analytes particularly present in physiological fluids and other suitable liquefied physiological samples either naturally or in consequence of previous administration. The method provided is particularly useful for the quantitative determination of low concentrations of specific protein or nucleic acid molecules, antigenic substances, receptors and their ligands without any phase separation procedure. The subject method is also useful in quantitative, automated analysis of any particular analyte having at least one natural or artificial specific binding pair.

The assay method applies specially separated space compartments.

The compartments may be separated by a membrane, or separator layer made of a suitable porous material like nitrocellulose, cellulose acetate and other cellulose derivatives, agarose, polyacrylamide and other synthetic polymers. The average pore size of such a membrane or separator layer is comparable to the size of the specific molecules and complexes applied in the assay. The pores are permeable for all assay components termed "small" but impermeable for "large" assay components.

The Modifier Compartment may be a complex formed entirely of the inner volumes of discrete solid porous particles (preferably microparticles) present in the assay medium, having a definite average pore size. Such particles are available commercially (dextran, agarose, polyacrylamide, glass beads, etc) or can also be produced from any porous or gel forming material including alginic acid, pectin, polygalacturonic acid and the like. The assay medium is preferably capable of communicating between the inner and outer spaces of such particles.

Alternatively, the Modifier Compartment may be a complex formed entirely of the inner volumes of microcapsules (or capsules) present in the assay medium, made of a polyanionic and a polycationic substance, e.g. an alginic acid core covered with chitosan, poly-L-lysine or any other polycationic substance, or a chitosan core covered with alginic acid, dextran sulphate or any other polyanionic substance. The inner core can also be liquified after the capsule has been made (liquid core capsules).

The assay method also applies members of a specific binding pair.

One member of the specific binding pair is the analyte, e.g. a nucleic acid or protein molecule or any antigenic substance or other ligand of practical significance. In some preferred realizations of the present invention, the presence or absence of the analyte molecule in a physiological sample, or changes in the concentration of the analyte in such a sample, or changes in the concentration of the analyte in such a sample will be characteristic of a physiological state, a disease, disorder or health condition, or the efficiency of a treatment. In other preferred realizations of the subject method the presence and concentration of the analyte in the assayed sample will be characteristic of a forensic or environmental pollution case or a scientific experiment. Without limitation, examples of analytes include protein such as protein hormones, soluble and cell surface receptors. serum proteins, tumor markers, enzymes, especially isoenzyme, enzyme inhibitors, antibodies, polymorphic, e.g. histocompatibility complex proteins, microbial proteins, viral proteins, proteins of living organisms occurring in the environment, proteins characteristic of any environmental pollution or forensic identification case and the like; nucleic acids such as viral and microbial nucleic acids, especially those of human and animal pathogens, environmentally important microbes, indicator organisms specific human, animal or plant nucleic acids such as oncogenies and genes of inheritable disorders, polymorphic nucleic acids, nucleic acid molecules characteristic of any species or a group of species useful in forensic and other identification cases and the like; small molecules such as steroid hormones, small peptide hormones, ligands of other practically important receptors, metabolites, amines, lipids, pharmaceuticals, drugs, toxic and physiologically active substances, environmental pollutants and the like.

The analyte is usually dissolved in the sample liquid, or may be solubilized by adding a suitable liquid. Alternatively, the analyte may be a particulate matter suspended in the sample liquid.

The other member of the specific binding pair is applied in the assay method as the binding reagent. This molecule is capable of specifically binding to the analyte and its analogues.

Preferably, the binding reagent is an antibody, produced in an animal or a human and purified from the body fluids of this mammal including humans, or from a cell culture derived from an animal or a human, or from microbial cells transformed with the nucleic acid coding for the antibody. The antibody may be used as a native molecule, or a fragment of the antibody molecule still capable of binding the analyte (Fab or F(ab)$_2$ or smaller fragments). Methods of obtaining such antibody molecules are obvious for those trained in the art, and can be found in textbooks and manuals like Lange, D. (ed.): Antibodies. A Laboratory Manual. Cold Spring Harbor Laboratory 1988 incorporated herein by reference.

The binding reagent may also be a DNA or RNA probe molecule. This probe molecule can be isolated from viral particles, microbial cells, or eukaryotic cells, or more preferably synthesized chemically or enzymatically. The probe can be a single stranded or double stranded nucleic acid molecule. The length of the probe will be in the range of 8 - 1,000 bases, preferably between 15 - 30 bases. Methods for obtaining such nucleic acid molecules are obvious for those trained in the art, and can be found in laboratory manuals like Ausubel, F.M., et al (eds): Current Protocols in Molecular Biology incorporated herein by this reference.

The binding reagent may also be a protein capable of specifically binding to a particular nucleic acid molecule (eg. repressor or activator molecules and other DNA and RNA binding proteins).

For other applications, the binding reagent may be a receptor molecule capable of specifically binding a hormone or a ligand. Alternatively, the binding reagent is a hormone or a ligand capable of specifically binding to a receptor.

In yet another application, the binding reagent may be a specific binding protein capable of binding a coenzyme, a metabolite, an enzyme inhibitor or any other compound. Examples of such binding proteins are avidin and streptavidin capable of binding biotin, and intrinsic factor capable of binding vitamin B$_{12}$ (cyanocobalamin). Alternatively, the binding reagent can be a coenzyme, a metabolite, an enzyme inhibitor or any other compound capable of binding a protein.

The assay method applies a label coupled to the binding reagent.

Preferably, the label is an enzyme. Usually, this enzyme produces an easily measurable signal. Preferably, this enzyme produces a change in the concentration of an optically or electrochemically active (fluorescent, luminescent, colored or charged) substance. Examples of such enzyme include among others oxidoreductases such as lactic acid dehydrogenase, alcohol dehydrogenase, peroxidase, catalase, glucose oxidase, diaphorase, glucose-6-phosphate dehydrogenase and the like; transferases hydrolases such as alkaline phosphatase, galactosidase, glucoronidase, glucose phosphates, uricase and the like; lyases such as pyruvate decarboxylase, glutamate decarboxylase and the like; isomerases; ligases such as glutamine synthetase, NAD synthetase and the like.

Such enzymes can be conjugated to the binding reagent molecules by a variety of methods, known for those trained in the art.

More preferably, this enzyme activates one or more other enzymes, inactive forms of coenzymes, or a nonenzymatic catalytic or other amplification mechanism. Examples of such amplification systems include zymogenic systems such as trypsinogen - trypsin, chymotrypsinogen - chymotrypsin, trypsin blood clotting cascades, complement system cascades and the like; coenzyme mediated systems such as galactosidase containing liposomes - lipase and the like.

(Such enzymatic amplification systems are described by Bates, Trends in Biotechnology July 1987, 204 - 209 incorporated herein by this reference).

Electronic amplification of an electrochemical or photochemical signal:

The label may be an optically or electrochemically active substance. This substance may be colored, or may be capable of emitting light or catalyzing the emission of light (luminescence), may be capable of emitting or quenching fluorescence, or may be able to significantly change the electric properties of the assay medium.

For another application, the label may be a radioactive substance emitting alpha, beta or gamma radiation.

The assay method may apply in the Modification Compartment specific signal modifier substances or binding substances.

When the label is an enzyme, the modifier substance can be an enzyme inhibitor, or an enzyme activator or a coenzyme. Examples of such substances are shown in Table I.

## TABLE I

### Enzyme inhibitors:                                              Enzyme:

| | |
|---|---|
| trypsin inhibitor from soybean | trypsin |
| trypsin inhibitor from bovine pancreas | trypsin |
| α-2-macroglobulin | trypsin |
| | chymotrypsin |
| amylase inhibitor from Phaseolus vulgaris | amylase |
| hirudin | thrombin |
| chelators | galactosidase |
| | amylase |

### Enzyme activators

Coenzymes:
NAD, NADP                                                        dehydrogenases
biotin                                          carbonic acid dehydrogenases
cyanocobalamine
ATP                                                                    kinases

Other activators:
metal ions                                                     metalloproteinases
special proteases                                                       zymogens
special phosphorylases                               inactive phosphoenzymes
inactive enzyme subunit                                       inactive enzyme
(alpha-peptide)                                       (mutant beta-galactosidase)

When the label is an enzyme, the modifier substance can be a protease.

When the binding reagent is an antibody, the binding substance may be an anti-immunoglobulin antibody, or a protein capable of binding immunoglobulins, for example protein A of Staphylococcus aureus.

When the binding reagent is a nucleic acid probe, the binding substance may be a nucleic acid molecule complementary to a part of the probe.

When the inner volume of porous particles are applied as the Modifier Compartment, assay components to be involved in this compartment are preferably covalently bound to the inner surface of said particles. Modifier or binding substances bound to the outer surface of said particles during the binding procedure are preferably eliminated or inactivated with the help of a macromolecular or immobilized agent (eg. an immobilized protease can digest protein substances from the outer surface of said particles).

When the inner volume of porous microcapsules are used as the Modifier Compartment, assay components to be involved in this compartment are preferably made macromolecular (eg. by covalent attachment to a macromolecular carrier) and incorporated in the capsules while preparing them.

The assay components to be included in the reaction can either be made macromolecular or can be bound to particles, or any material incapable of entering the Modifier Compartment. Methods for coupling assay compartments to other molecules or surfaces are known to those trained in the art, and can be found in textbooks and laboratory manuals like volume 104 of the series Methods in Enzymology, or Dean, Johnson and Middle (eds.): Affinity Chromatography. IRL Press, Oxford, 1985 incorporated herein by this reference.

The assay method may apply additional components necessary for signal generation in the Sample Compartment.

When the Modifier Compartment contains only binding but not modifying substances and the label is an enzyme, the additional components to be applied can be a macromolecular substrate, a macromolecular activator or a macromolecular coenzyme, all incapable of entering the Modifier Compartment. These additional components may be macromolecular by nature, or can be rendered macromolecular by coupling them to a macromolecular carrier like dextran, starch, or any other natural or artificial polymer.

The assay method may apply a special force or pressure to facilitate the diffusion of certain assay components during the course of the assay procedure.

When the Modifier Compartment is the inner volume of porous particles, during the course of the assay procedure an osmotic pressure may be applied to facilitate the diffusion of small assay components from the Sample Compartment to the Modifier Compartment by adding a high concentration of large molecules (e.g. dextran) to the Modifier Compartment.

METHOD

The subject assay in general is carried out as follows:

The analyte can be dissolved in a suitably buffered liquid or liquified sample which is added to the Sample Compartment. The suitably labelled binding reagent (BR-L) is also added to this compartment prior to, together with or after the addition of the sample. The liquid sample and the Sample Compartment may also contain other previously added assay components. Assay components applied in the Sample Compartment are altogether suitable to create optimal conditions for the specific binding reaction between BR-L molecules and analyte, analyte analog molecules, analyte second binding reagent molecules, and to minimize undesirable nonspecific binding reactions between other assay components.

During incubation at a predetermined temperature, usually about 0 to about 100°C, preferably about 20 to about 60°C, more preferably about 20 to about 37°C for a predetermined time, usually about 1 minute to about 24 hours, preferably about 5 minutes to about 2 hours, more preferably about 5 minutes to about 20 minutes, the binding reaction takes place, and the assay components distribute over the entire volume available for them. Assay components defined as "small" can pass the inter-compartment barrier, while the assay components defined as "large" remain in the compartment where they were added. All free and complexed BR-L molecules which water the Modifier Compartment are entrapped there and/or their signal producing capability is significantly altered. After the specific binding reaction takes place in a sufficient measure, a special force or pressure (eg. centrifugal or electric force; osmotic, capillary or mechanical pressure, hydration or solvation energy of a special material, etc.) may be applied to facilitate the diffusion of some or all small assay components from the Sample Compartment to the Modifier Compartment.

After this period of time, optionally further assay components instrumental in signal generation and detection (e.g. enzyme substrates, signal amplifying agents, etc.) are added, and after an optional further incubation step at a predetermined temperature for a predetermined time, the signal generated in one or both separated compartments is measured in a suitable instrument. Because of the applied special modifying reagents, assay arrangements and/or detection systems, the measured reagents, assay arrangements and/or detection systems, the measured signal is always induced solely by analyte or analyte analog bound BR-L molecules and its is strictly related to the amount of analyte originally present in the sample.

Without limiting the scope of the present invention, preferred realizations are described as follows.

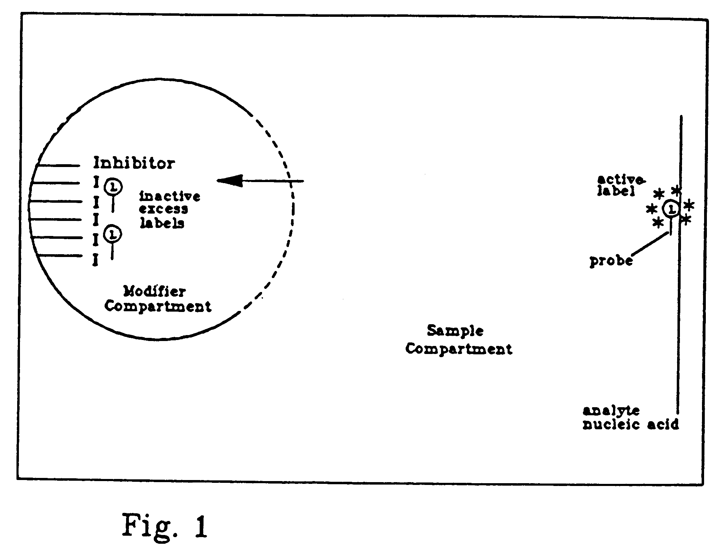

As illustrated in Fig. 1, the invention is used for the detection of large nucleic acid analyte molecules. The Modifier Compartment herein is the complex entirety of the inner volumes of some discrete porous particles or capsules wetted by the assay medium, added to the Sample Compartment before or during the assay. An effective enzyme inhibitor is incorporated in the particles and the probe-label (BR-L) conjugates to the Sample Compartment (which is the whole reaction vessel herein), a certain portion of the added BR-L conjugates will bind to the large analyte molecules thus becoming unable to enter the Modifier Compartment. After a certain period of incubation, all excess BR-L conjugates enter the Modifier Compartment where their enzymatic activity is inhibited. Only analyte bound BR-L conjugates in the Sample Compartment will then be able to produce a measurable signal, which signal is thus related to the amount of the analyte present in the assayed sample.

Based on this realization, the following materials may be substituted to further demonstrate the concept of Figure 1:

| Analyte | BR-L | Additional agent required in Sample Compartment |
|---------|------|-------------------------------------------------|
| large antigen (eg. large protein like ferritin) | labelled antibody | none |
| small bivalent antigen (eg. small proteins insulin, albumin,etc) | labelled antibody | second antibody attached to any material incapable of entering the Modifier Compartment |
| small nucleic acid binding protein (eg. labda-repressor | labelled antibody | large target nucleic acid molecule (eg. phage lambda) |

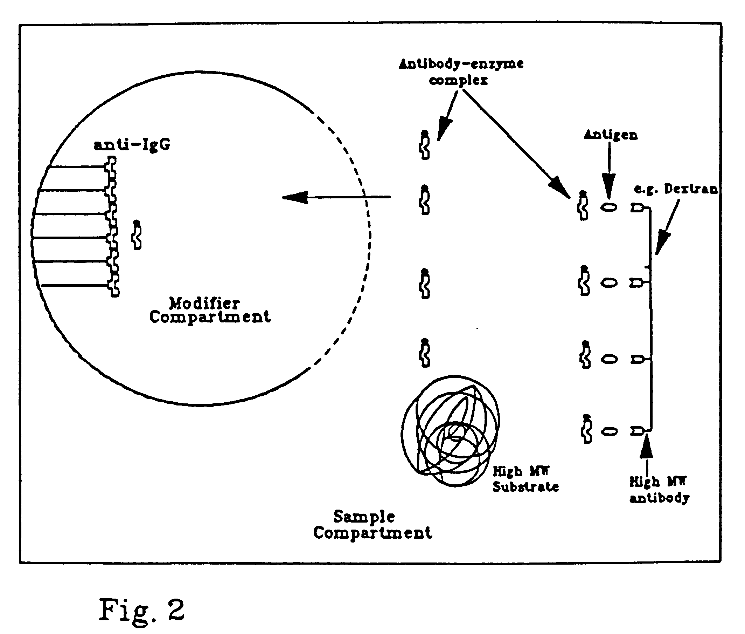

As illustrated in Fig. 2, the invention is used for the detection of small bivalent antigenic analyte molecules. The Modifier Compartment herein is again the complex entirety of the inner volumes of some discrete porous particles or capsules wetted by the assay medium, added to the Sample Compartment before or during the assay. An effective binding substance is incorporated in the particles and the applied label is an enzyme. After adding the sample, excess macromolecular antibody (second binding reagent, SBR) and excess enzyme labelled antibody (BR-L) molecules to the Sample Compartment, the analyte molecules bind to the macromolecular antibody and the enzyme labelled antibody at the same time, thus preventing the bound BR-L conjugates from being entrapped in the Modifier Compartment, where the excess BR-L conjugates are entrapped. After adding a macromolecular substrate incapable of entering the Modifier Compartment, enzymatic signal is generated only by the analyte bound BR-L conjugates, and this signal is thus strictly related to the amount of the analyte present in the assayed sample.

Based on this realization, the following materials may be substituted to further demonstrate the concept of Fig. 2.

| Analyte | BR-L | Additional agent required in the Single Compartment |
|---------|------|-----------------------------------------------------|
| large nucleic acid molecule | labelled probe | none |
| small nucleic acid binding protein (eg. lambda-repressor | labelled antibody | large target nucleic acid molecule (eg. phage lambda) |
| large antigen (eg. large particles like ferritin) | labelled antibody | none |

In an assay method used to detect nucleic acid analytes (nucleic acid probe assays), the Modifier Compartment contains an efficient inhibitor of signal generation (eg. a fluorescence quencher or light absorbing substances) and efficient binding substances incapable of leaving the Modifier Compartment. The sample is added to the Sample Compartment where, during the incubation period the large analyte nucleic acid molecules conjugated to a labe, e.f. a fluorescent or luminescent atom or molecule. These complexes will be large enough to be unable to enter the Modifier Compartment, so the label molecules contained in these complexes retain their full signal emitting activity. The excess probe-label conjugate molecules not bound to the analyte molecules enter the Modifier Compartment where they get entrapped, and therefore their signal emitting activity is significantly diminished. The signal emitted by the analyte bound probe-label conjugates, related to the amount of the analyte in the sample, is measured after adding the necessary assay components.

When used to detect nucleic acid analytes (nucleic acid probe assays), the Modifier Compartment contains an efficient binding substance capable of binding the probe-label conjugate, either through the probe or the label molecules, or both. The sample is added to the Sample Compartment where, during the incubation period the large analyte nucleic acid molecules, if present, bind to the probe nucleic acid molecules conjugated to a label. These complexes will be large enough to be unable to enter the Modifier Compartment. The excess probe-label conjugate molecules not bound to analyte molecules enter the Modifier Compartment where they bind to the binding substance molecules. At least one further assay component, necessary for signal generation and unable to enter the Modifier Compartment, is added. Thus, the excess probe-label conjugate molecules entrapped in the Modifier Compartment will be unable to generate a signal. The signal generated by the label molecules of the analyte bound probe-label conjugates, related to the amount of analyte in the sample, is measured.

In a method used to detect large antigenic substances (immunoassays), the Modifier Compartment contains an efficient inhibitor of signal generation (eg. a fluorescent quencher or light absorbing substances) and efficient binding substances incapable of leaving the Modifier Compartment. The sample is added to the Sample Compartment where, during the incubation period, the large antigenic analyte molecules, if present, bind to the antibody molecules conjugated to a label, eg. a fluorescent or luminescent atom or molecule. These complexes will be large enough to be unable to enter the Modifier Compartment, so the label molecules contained in these complexes retain their full signal emitting activity. The excess antibody-label conjugate molecules not bound to analyte molecules enter the Modifier Compartment where they get entrapped, and therefore their signal emitting activity is significantly diminished. The signal emitted by the analyte bound antibody-label conjugates, related to the amount of analyte in the sample, is measured after adding the necessary assay components.

In a method used to detect large antigenic substances (immunoassays), the Modifier Compartment contains an efficient binding substance capable of binding the antibody-label conjugate, either through the antibody or the label molecules, or both. The sample is added to the Sample Compartment where, during the incubation period the large analyte molecules, if present, bind to the antibody molecules conjugated to a label. These complexes will be large enough to be unable to enter the Modifier Compartment where they bind to the binding substance molecules. At least one further assay component, necessary for signal generation and unable to enter the Modifier Compartment, is added. Thus, the excess antibody-label conjugate molecules entrapped in the Modifier Compartment will be unable to generate a signal. The signal generated by the label molecules of the analyte bound antibody-label conjugates, related to the amount of analyte in the sample, is measured.

In an assay method used to detect small antigenic substance with at least two simultaneously available antibody binding sites (sandwich immunoassays), the small antigenic molecules are rendered large by binding to an antibody, different from the binding reagent, coupled to a large molecule. This binding may take place before adding the sample to the Sample Compartment by mixing the sample with a liquid containing the antibody coupled to a large molecule, or, preferably, after adding the sample to the Sample Compartment. All subsequent steps are identical to those described in any of the above immunoassays realizations of the subject invention.

In an assay method used to detect small antigenic substances with only one available antibody binding site (competitive immunoassays), the antibody-level conjugate is preferably preincubated with the sample, during which incubation they bind to the analyte molecules. These complexes will be able to enter the Modifier Compartment. The excess antibody-label conjugate molecules not bound to analyte molecules, when applied in the Sample Compartment, bind to an analogue of the analyte coupled to a large molecule, provided in the Sample Compartment. These complexes will be unable to enter the Modifier Compartment. The Modifier Compartment preferably contains an activator. The small analyte-antibody-label complexes which entered the Modifier Compartment will emit a signal, while the large analyte analogue excess antibody-label complexes will not. The signal emitted by the analyte bound antibody-label conjugated, related to the amount of analyte in the sample, is measured after adding the necessary assay components.

## EXAMPLES

### Example 1: Preparation of 6% polyacrylamide - soybean trypsin inhibitor particles.

Soybean trypsin inhibitor (SBTI) was coupled to acrylic acid, and polymerized into an acrylamide gel. Excess SBTI was removed by electrophoresis. The gel was cut into particles. These particles served as a Modifier Compartment containing an enzyme inhibitor.

Acrylic derivation of SBTI - 5 mg SBTI was dissolved in 1 mL 0.1 M Sodium Bicarbonate, pH 8. 100 $\mu$moles of acrylic chloride in 1 mL diethyl ether was added and stirred overnight. Next day the pH was lowered to 3 and excess acrylic acid was extracted with ethyl acetate.

Polymerization - The following reagents were mixed and left for one hour to polymerize:

1 mL 30% acrylamide + 0.8% bis-acrylamide

0.5 mL 0.2 M Tris-HC1, pH 8.0

0.5 mL acryl-SBTI

0.1 mL 10% TEMED

2.9 mL Distilled water

0.1 mL 10% ammonium persulphate

Electrophoresis - The gel was put in an electrophoresis chamber containing 0.1 M Tris-Hc1, pH 8.3 buffer, and electrophoresed for 1 hour at 100 Volts.

Particle formation - The gel was cut into smaller pieces by a scalpel, placed in 100 mL of 0.1 M Tris-HC1, pH 8.0 in a blender that was used for 30 seconds. The resulting particles were washed 3 times with 0.1 M Tris-HC1 pH 8.0 containing 0.02 M $CaCl_2$.

Quality control of the particles - Particle bound SBTI was estimated by making a serial dilution of the particles and a standard SBTI solution, in 0.1 mL Tris-HC1 buffer containing 0.02 M $CaCl_2$ (assay buffer). To each of these samples 0.5 pmoles of trypsin were added, and incubated for 15 minutes at room temperature. The 10 μL of 10 mg/mL z-Gly-Pro-Arg-p-nitro-anilide substrate was added, and the resulting color was measured kinetically at 405 nm in a Molecular Devices reader.

SBTI release from the particles was estimated by washing the particles in assay buffer, leaving them to stand overnight at 4°C, and measuring the SBTI content of the supernatant as described above.

Results:

Of the total SBTI activity added, 15% was found in the electrophoresis buffer. A further 15% was found in the blender buffer, and 1% was retained in the particles. SBTI release from the particles was less than 0.1%.

**Example 2: Quantitative detection of ferritin-avidin using polyacrylamide-SBTI particles.**

A serial dilution of ferritin-avidin analyte, and ferritin as a control substance (similar in structure to the analyte), in 0.1 M Tris HC1 pH 8.0 containing 0.02 M $CACl_2$ (assay buffer), from 8.5 to 0.033 pmoles/well was made on a microtiter plate. 1.0 pmole trypsin-biotin as the BR-L conjugate was added to each of these samples in 0.01 mL assay buffer. After 10 minutes. 0.2 mL SBTI containing polyacrylamide particles were added. The interior of these particles served as the Modifier Compartment. During an incubation period of 1 hour, free BR-L conjugates migrated in the Modifier Compartment and their trypsin activity was inhibited by SBTI. Trypsin activity of the bound BR-L conjugates remaining in the bulk of the liquid was measured after 1 hour by adding to each well 10 μl of 10 mg/mL a-Gly-Pro-Arg-p-nitro-anilide substrate, and measuring the resulting color kinetically at 405 nm in a Molecular device reader.

Results:

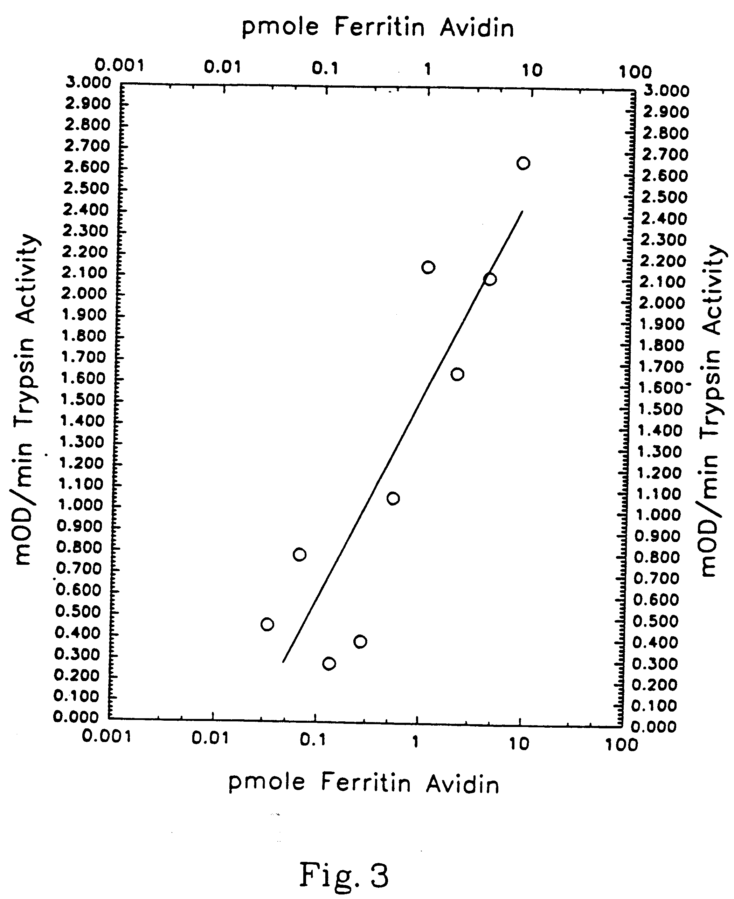

Fig. 3 shows the measured trypsin activity (rate of color change) as a function of ferritin-avidin concentration. As evident from Fig. 3, the measured signal is proportional to the concentration of ferritin-avidin analyte.

## Claims

1. A method for determining the amount of analyte in a sample which comprises:
   (a) providing a first assay compartment and a second assay compartment separated from the first assay compartment by a barrier permeable to a signal providing binding reagent-label conjugate, the barrier e.g. being a porous membrane or gel;
   (b) combining an analyte containing sample and binding reagent-label conjugate capable complex of binding to the analyte to form alone or in combination with a second binding reagent a specific binding pair complex of a size sufficiently large to prevent passage through the barrier, said binding reagent-label conjugate being provided in an excess of the amount required to bind with all of the analyte;
   (c) incubating the mixture to allow the analyte to bind to the binding reagent-label conjugate to form the specific binding pair comprising the analyte and the binding reagent-label conjugate and for unbound binding reagent-label conjugate to pass through the barrier into the second compartment;
   (d) entrapping and modifying the unbound binding reagent-label conjugate in the second compartment to enable detection of a signal from specific binding pair as a measure of the amount of analyte bound in the specific binding pair; and
   (e) measuring the detectable signal.

2. A method as claimed in claim 1 in which a second binding reagent which binds to the specific binding pair and is added to the first assay compartment.

3. A method as claimed in claim 2 in which the specific binding pair is a molecular size sufficiently small to enter the second compartment and where the second binding reagent is bound to the specific binding pair in the first compartment to prevent the specific binding pair from entering into the second compartment.

4.  A method as claimed in claim 1 in which the second compartment is a self contained enclosure comprising the barrier which is physically separable from liquid contained in the first compartment and in which unbound binding reagent-label conjugate contained in the second compartment is prevented from producing a detectable signal.

5.  A method as claimed in claim 1 to 4 in which the specific binding pair formed by the binding reagent-label conjugate and analyte are sufficiently small to pass through the barrier and are bound in the first compartment to a second binding reagent through the analyte to prevent transport of the analyte into the second compartment.

6.  A method as claimed in any of the preceding claims in which the binding reagent of the binding reagent-label conjugate is selected from the group consisting of antibodies, antigens, nucleic acids, non-nucleic acids, receptors, ligands and proteins capable of specifically binding to an analyte selected from the group consisting of nucleic acids, non-nucleic acids, proteins, antigens, and antibodies, ligands and receptors.

7.  A method as claimed in any of the preceding claims, wherein said label of the binding reagent-label conjugate is selected from an enzyme, a substance capable of binding an enzyme, a conjugate and a complex having a detectable enzymatic activity.

8.  A method as claimed in any of the preceding claims, wherein said detectable signal is in a form selected from color, fluorescence liminescence, conductivity, a radiation or a combination thereof.

9.  A method for determining the amount of analyte contained in a liquid sample, which comprises:
    (a) providing a first assay compartment and a second assay compartment separated from the first assay compartment by a barrier permeable to conjugates less than a predetermined size;
    (b) combining a liquid sample containing an analyte to be measured and a binding reagent-label conjugate which is capable of signal production, passing through the barrier and binding to the analyte to form with the analyte a alone or in combination with a second binding reagent a complex which is a signal providing specific binding pair of a size sufficiently large to prevent passage through the barrier, such binding reagent-label conjugate being provided in an amount in excess of the amount required to bind with all of the analyte in the liquid sample;
    (c) incubating the mixture for a time sufficient to allow a signal producing specific binding pair to form, said specific binding pair being of a size too large to pass through the barrier and for unbound binding reagent-label conjugate to pass through the barrier into the second compartment;
    (d) retaining and disabling the unbound binding reagent-label conjugate in the second compartment from interfering with the signal produced by the specific binding pair contained in the first compartment; and
    (e) measuring the signal produced by the specific binding pair contained in the first compartment as a measure of analyte content of the sample.

10. A method for determining the amount of analyte contained in a liquid sample, which comprises:
    (a) providing an assay means comprising a first compartment and a second modifier compartment formed of discrete porous particles, e.g. microcapsules, dispensable in the liquid contained in the first compartment, said particles having a pore size which enables molecular conjugates and complexes less than a predetermined size to enter such pores;
    (b) combining the liquid sample with a binding reagent-label conjugate which is capable of signal production, passing into the porous particles and of binding to the analyte to form a signal providing specific binding pair complex alone or in combination with a second binding reagent, said signal providing specific binding pair being of size sufficiently great to prevent passage into the porous particles, such binding reagent-label being provided in an excess of the amount required to bind with the analyte;
    (c) incubating the mixture to allow the analyte to bind to the binding reagent-label and form the signal producing specific binding pair and for unbound binding reagent-label conjugate to pass into the pores of porous particles;
    (d) preventing the binding reagent-label conjugate in the porous particles from producing a detectable signal; and
    (e) measuring the signal produced by the signal producing specific binding pair as a measure of analyte content of the liquid sample.

11. A method for determining the amount of analyte contained in a liquid sample, which comprises;

(a) providing a first assay compartment and a second assay compartment separated from the first assay compartment by a barrier permeable to select binding reagent-label conjugate;

(b) combining the liquid sample containing an analyte selected from the group consisting of proteins, nucleic acids and molecules which are a measure of physiological condition with a binding reagent-label conjugate compatible with the liquid sample which binding reagent-label conjugate is a molecule of a size capable of passing through the barrier and of binding to the analyte alone or in combination with a second binding reagent to form a signal producing specific binding pair complex of a size sufficiently large to prevent passage through the barrier, such binding reagent- label conjugate being provided in an excess of the amount required to bind with all of the analyte;

(c) incubating the mixture to allow without phase separation, the analyte to bind to the binding reagent-label conjugate to form a signal producing specific binding pair and for unbound binding reagent-label to permeate through the barrier into the second assay compartment for retention therein, said signal producing specific binding pair providing a detectable signal, the presence and intensity of which is a measure of the presence and concentration of the analyte in the liquid sample; and

(d) selectively measuring the signal of the specific binding pair to the exclusion of any signal attributable to binding reagent-label conjugate not bound to the analyte and contained in the second assay compartment.

12. A method for determining the amount of analyte in a sample which comprises:

(a) providing a first assay compartment and a second assay compartment separated from the first assay compartment by a barrier permeable to a signal providing binding reagent-label conjugate;

(b) combining the analyte containing sample and a predetermined amount of binding reagent-label conjugate capable of binding to the analyte to form a specific binding pair of a size sufficiently large to prevent passage through the barrier, said signal producing binding reagent-label conjugate being provided in an excess of the amount required to bind with all of the analyte;

(c) incubating the mixture to allow the analyte to bind to the binding reagent-label conjugate to form the specific binding pair and for unbound signal producing binding reagent-label conjugate to pass through the barrier into the second compartment;

(d) retaining the signal producing unbound binding reagent-label in the second compartment; signal which is a measure of the amount of analyte bound to the binding reagent-label;

(e) measuring the detectable signal produced by the unbound signal producing binding reagent-label conjugate for the second compartment as an indirect measure of the amount of analyte added with the sample to the first compartment, and optionally an analyte analogue is provided to enhance the first assay compartment to combine with the analyte and binding reagent-label conjugate, and optionally a second binding reagent is provided to the first assay compartment to combine with the analyte and binding reagent-label conjugate.

Fig. 1

Fig. 2

Fig. 3